# EUROPÄISCHE PATENTSCHRIFT

(11) **EP 3 568 226 B1**
(45) Veröffentlichungstag und Bekanntmachung des Hinweises auf die Patenterteilung: **13.01.2021**
(21) Anmeldenummer: 18702896.4
(22) Anmeldetag: 15.01.2018
(51) Int. Cl.: B01D 53/04, A61B 18/00, B01D 53/26

(54) **VORRICHTUNG ZUR ABTRENNUNG VON RAUCHGASPARTIKELN BEI DER LAPAROSKOPIE**
DEVICE FOR THE SEPARATION OF FLUE GAS PARTICLES IN LAPAROSCOPY
DISPOSITIF DE SÉPARATION DE PARTICULES DE GAZ DE FUMÉE EN LAPAROSCOPIE

(30) Priorität: 13.01.2017 DE 102017000219
(43) Veröffentlichungstag der Anmeldung: 20.11.2019
(73) Patentinhaber: W.O.M. World of Medicine GmbH, 10587 Berlin (DE)
(72) Erfinder: LANGEN, Fabian, 10407 Berlin (DE); JÜLG, Peter, 79098 Freiburg (DE)
(74) Vertreter: Seuss, Thomas
(86) Internationale Anmeldenummer: PCT/DE2018/000007
(87) Internationale Veröffentlichungsnummer: WO 2018/130243

(56) Entgegenhaltungen:
- DE-A1- 2 947 737
- US-A- 5 460 602
- US-A- 5 968 032
- US-A1- 2005 172 589
- US-A1- 2011 041 468
- US-A1- 2014 165 842
- US-B1- 6 524 307
- US-B1- 6 592 543

## Beschreibung

Gegenstand der vorliegenden Erfindung ist eine Vorrichtung zur Abtrennung von Rauchgaspartikeln, Flüssigkeitströpfchen und Feuchtigkeit bei der Absaugung chirurgischer Gase im Rahmen laparoskopischer Eingriffe.

Minimal-invasive Operationstechniken erfreuen sich steigender Beliebtheit. Beim intraoperativen Einsatz von Laserskalpellen oder anderer Elektro- und Ultraschallchirurgischer Instrumente im Rahmen minimal-invasiver Eingriffe, z.B. in der Laparoskopie kommt es häufig zur Rauchgasentwicklung innerhalb des Patientenkörpers. Derartige Rauchgase enthalten ein komplexes Gemisch partikel-, tröpfchen- und gasförmiger Komponenten. Derartige Rauchgase dürfen bereits aus Sicherheitsgründen nicht ungefiltert in den Operationsraum entlassen werden. Moderne Insufflatoren weisen häufig entsprechende Abluftschläuche und dazugehörige Filtereinrichtungen auf, mit denen die im Körper des Patienten entstehenden Rauchgase gefiltert werden können. Es handelt sich dabei in der Regel um Faserfilter oder Membranen, welche Tröpfchen und Partikel filtern. Derartige Filter haben verschiedene Nachteile: Membranen setzen sich relativ leicht zu, Faserfilter benötigen eine geringe Strömungsgeschwindigkeit, um optimal filtern zu können.

Es besteht daher Bedarf, eine Vorrichtung zu entwickeln, die Tröpfchen, Feuchtigkeit und Partikel aus intraoperativ entstandenen Rauchgasen wirkungsvoll zurückhält, ohne mit den beschriebenen Nachteilen behaftet zu sein. Aus dem Stand der Technik sind in diesem Zusammenhang folgende Druckschriften bekannt:
1. US 2011/041468 A1
2. US 2014/165842 A1
3. US 6 592 543 B1
4. US 5 968 032 A
5. US 6 524 307 B1
6. US 5 460 602 A
7. DE 29 47 737 A1

Aus großtechnischen Anlagen sind ferner sogenannte Zyklonabscheider bekannt, die die Aufgabe haben, größere Partikel oder Tropfen aus Gasströmen abzuscheiden. Beispiele hierfür sind in den Druckschriften US 4,255,174, US 2013/0152525 A1, US 2005/0172589 und EP2832449A1 beschrieben.

Die vorliegende Erfindung stellt eine Vorrichtung zur Verfügung, die trotz ihrer Einfachheit die oben genannten Probleme löst. Es handelt sich hierbei um eine im Wesentlichen rohrförmig gestaltete Einheit, die beispielsweise nach Art eines Verbinders zwischen zwei Schlauchteilen angeordnet sein kann. Die röhrenförmige Vorrichtung weist in ihrem Inneren Leitschaufeln auf, die einem durchgeleiteten Gasstrom eine radiale Bewegungskomponente geben. Ein durch die erfindungsgemäße Vorrichtung geleiteter, im Wesentlichen laminarer oder turbulenter Gasstrom wird nach Passieren der Vorrichtung helix- oder spiralförmig weitergeleitet. Durch die entstehenden Zentrifugalkräfte werden Tröpfchen und Partikel an die Schlauchwand geführt, wo sie durch Adhäsionskräfte abgeschieden werden. Zusätzlich findet beim Durchströmen der Vorrichtung ein Druckabfall statt, der zu einer Kondensation von im Gas gelösten Flüssigkeiten und somit zu Kondensatbildung führt.

Gegenstand der Erfindung ist daher eine Vorrichtung zur Abscheidung von Rauchgaspartikeln, Flüssigkeitströpfchen und Feuchtigkeit aus abgesaugten chirurgischen Gasen im Rahmen laparaskopischer Eingriffe,
wobei die Gase eine einer Strömungsgeschwindigkeit von 3-20 l/min aufweisen,
mit einem Rohr der Länge I von 10-50 mm, wobei das Rohr einen Innendurchmesser dᵢ von 4-20 mm aufweist,
gekennzeichnet durch
einen axial gelegenen Strömungskörper K, der einen Spantquerschnitt von 0,5-6 mm aufweist,
mit zwei bis sechs Leitschaufeln L, die zwischen dem axial gelegenen Strömungskörper K und der Innenwand des Rohrs liegen und mit diesen starr verbunden sind, wobei die Leitschaufeln L helixförmig angeordnet sind, so dass sie dem durchströmenden Gas eine radiale Bewegungskomponente geben.

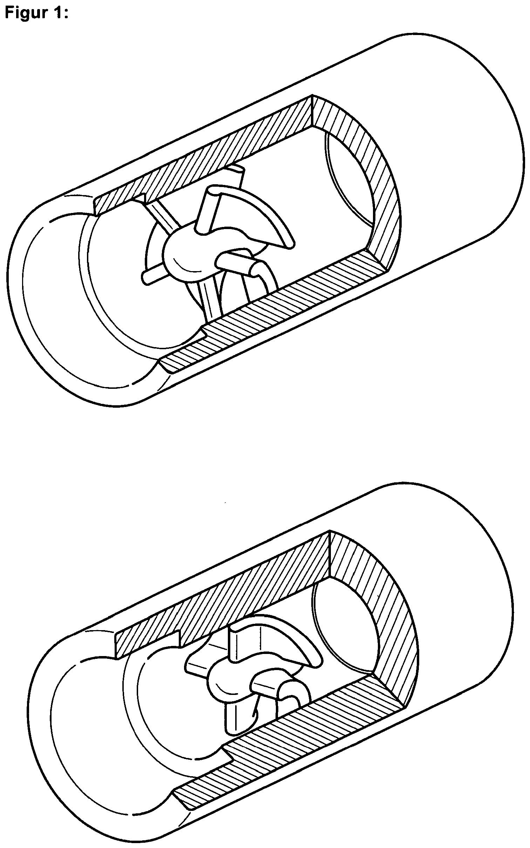

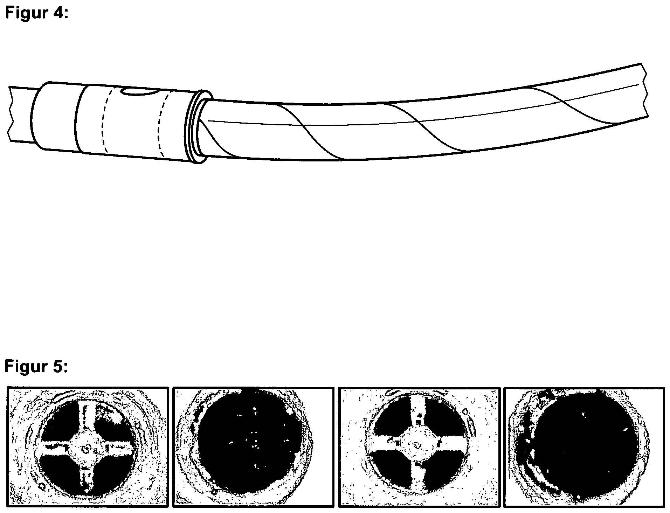

Figur 1 zeigt zwei Beispiele erfindungsgemäßer Vorrichtungen. In beiden Fällen ist die Vorrichtung so ausgestaltet, dass im Inneren der röhrenförmigen Vorrichtung ein axialer Strömungskörper mit 5 (obere Darstellung) bzw. 4 (untere Darstellung) Leitschaufeln angeordnet ist. Sowohl die Achse, wie auch die Leitschaufeln sind starr, letztere sind mit dem Außenzylinder verbunden. Beim Durchleiten von Rauchgasen durch die erfindungsgemäße Vorrichtung tritt eine Abscheidung von Flüssigkeit und Partikeln im nachfolgenden Schlauch auf (siehe Figur 4). Figur 4 zeigt auch das typische helixförmige Abscheidungsmuster im Schlauch. Da die spiralförmige Verwirbelung und somit die entstehenden Zentrifugalkräfte mit zunehmenden Abstand von der Vorrichtung abnehmen, tritt der größte Teil der Abscheidung vorrichtungsnah ein. Eine Teilabscheidung tritt bereits an der Austrittsöffnung der Vorrichtung auf (siehe Figur 5).

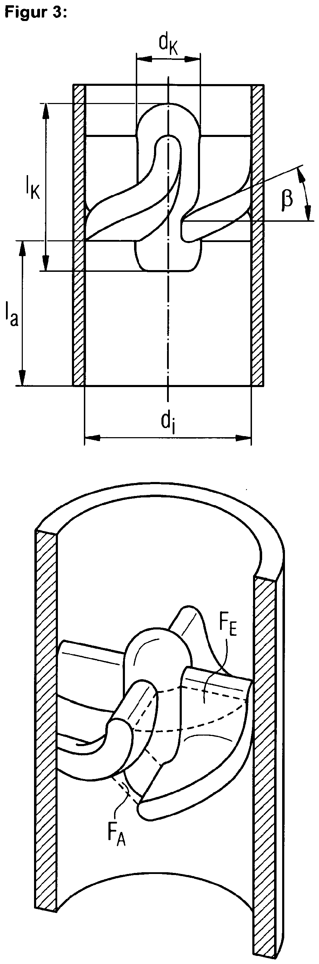

Figur 3 (oben) zeigt einen Querschnitt einer erfindungsgemäßen Vorrichtung. Anzahl und Geometrie der erfindungsgemäßen Vorrichtung sind sehr variabel. Im Allgemeinen wird der Innendurchmesser dᵢ der erfindungsgemäßen Vorrichtung im Bereich von 4 bis 20 mm liegen. Der Durchmesser des axialen Strömungskörpers (d_{K}) kann im Bereich von 0,5 - 6 mm, bevorzugt 1-4 mm, liegen. Erfahrungsgemäß werden gute Abscheideergebnisse beim Einsatz von 3 - 6 Leitschaufeln erreicht. Denkbar ist aber auch eine Ausgestaltung mit nur zwei helixförmigen Leitschaufeln oder mit mehr als 6 Schaufeln. Der Austrittwinkel β der Leitschaufeln sollte zwischen 15 und 30 Grad liegen. Hierbei sollte angemerkt werden, dass der Austrittswinkel β nicht über den ganzen Radius konstant sein muss, sondern variabel sein kann: So kann er beispielsweise 30 Grad beim Ansatz an den inneren Strömungskörper sein und im radialen Verlauf auf 20 Grad beim Auftreffen auf die Innenwand des Rohres fallen.

Figur 3 (unten) zeigt die sogenannte Schaufeleintrittsfläche F_{E} und die Schaufelaustrittsfläche F_{A}, welche für die Abscheidung eine Rolle spielen:Das Verhältnis S der Flächen F_{E} : F_{A} sollte zwischen 1 und 8, bevorzugt zwischen 2 und 5 liegen. Besonders bevorzugt ist ein Flächenverhältnis von 2,7 bis 3,3.

In speziellen Ausführungsformen kann auch nur eine einzige Leitschaufel ausreichen, um eine Abscheidung zu ermöglichen. In diesem Fall wird der Gasfluß durch die rohrförmige Vorrichtung durch eine Trennwand getrennt, die im Rohr helixförmig angeordnet ist. Um die erforderlichen Radialkräfte zu gewährleisten muss die helixförmige Trennwand eine Drehung von mindestens 180°, bevorzugt mindestens 270°, besonders bevorzugt mindestens 360° aufweisen.

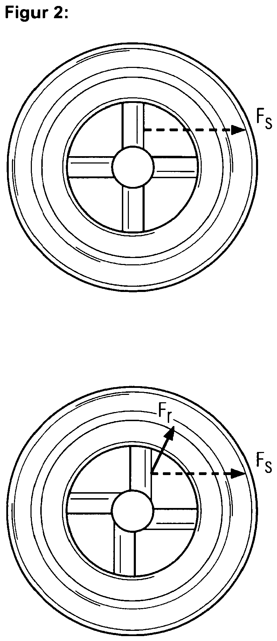

Auch für die Verbindung der Leitschaufeln mit dem axial angeordneten Strömungskörper gibt es unterschiedliche Geometrien: Die Verbindung kann orthogonal oder sekantenförmigsein, wie in Figur 2 am Beispiel von jeweils 4 angeordneten Leitschaufeln dargestellt. Bei sekantenförmiger Anordnung tritt zusätzlich zur senkrecht zu den Leitschaufeln wirkenden Kraft (Fₛ) ein weiterer radialer Kraftvektor (Fᵣ) auf, so dass eine verbesserte Abscheideleistung resultiert.

Für bevorzugte Ausführungsformen der Erfindung gilt:
1. Die Anzahl der Schaufeln sollte zwischen 3 und 5 liegen.
2. Das Verhältnis der Länge lₖzu Spantquerschnittdₖ des Axialkörpers sollte zwischen 2 und 3 liegen.
3. Das Verhältnis der Länge hinter den Leitschaufeln bis zum Gasaustritt la zum Spantquerschnittdₖsollte zwischen 2 und 4 liegen.
4. Das Verhältnis S der Schaufeleintrittsfläche F_{E}zur Schaufelaustrittsfläche F_{A}(siehe Figur 2) sollte zwischen 2,7 und 3,3 liegen.

An dieser Stelle sollte nochmals betont werden, dass die erfindungsgemäße Vorrichtung keine beweglichen Teile aufweist und daher in einfacher Weise hergestellt werden kann. Die Herstellung kann z.B. durch Spritzguss oder 3D-Druck erfolgen. Bei einer Ausführungsform der Schaufeln ohne Hinterschneidung kann die Herstellung per Spritzguss mit einem einfachen und kostengünstigen Auf-Zu-Werkzeug erfolgen. Eine weiter verbesserte Abscheideleistung wird erzielt, wenn die Schaufeln eine Hinterschneidung aufweisen. Zur Herstellung derartiger Vorrichtungen mit einer Hinterschneidung der Schaufeln muss bei der Spritzgussherstellung mit einem Spindeleinsatz gearbeitet werden.

Bei der Herstellung im Wege des 3D-Druckes können alle Ausführungsformen ohne besondere Schwierigkeiten realisiert werden.

Die erfindungsgemäße Vorrichtung kann auch in andere Bauteile medizintechnischer Vorrichtungen integriert sein wie z.B. in Filtergehäuse, Schlauchadapter oder Ähnliches. Die erfindungsgemäße Vorrichtung kann gewünschtenfalls auch mit anderen Filtereinrichtungen kombiniert werden. So kann der Rauchgasstrom beipielsweise erst durch eine erfindungsgemäße Vorrichtung geführt werden und anschließend durch andersartige Filter (Faserfilter, Membranfilter, Aktivkohlefilter o.ä.).

Der Abscheidegrad wird auch durch die Wandrauhheit der stromabwärtsgelegenen Schläuche und Rohre beeinflusst. Je höher die Rauhheit des Materials ist, desto besser üblicherweise ist die Abscheidung. Dem Fachmann ist bekannt, dass die Rauhheit durch Verwendung entsprechender Materialien, Anpassung der Oberflächengestaltung des Spritzgießwerkzeuges oder durch Oberfächenbeschichtungen verändert werden kann. Hydrophile Materialien und Beschichtungen verbessern naturgemäß insbesondere die Abscheidung flüssiger (tröpfchenförmiger) Rauchgaskomponenten.

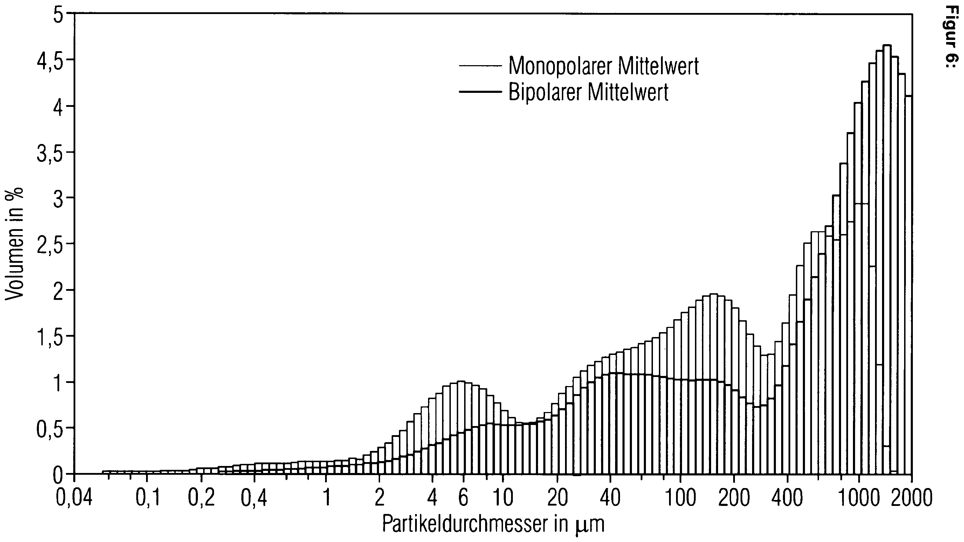

Figur 6 (entnommen aus: Farrugia, M.; Hussain, S. Y. et al. (2009): Particulate Matter Generated During Monopolar and Bipolar Hysteroscopic Human Uterine Tissue Vaporization, in: Journal of Minimally Invasive Gynecology, Jg. 16, Nr. 4, S. 458-464) zeigt die Partikelgrößenverteilung in typischen Rauchgasen in Form des Volumenanteils: Deutlich zu sehen ist, dass der überwiegende Teil der Partikel eine Größe zwischen 2 und 2000 Mikrometer aufweist.

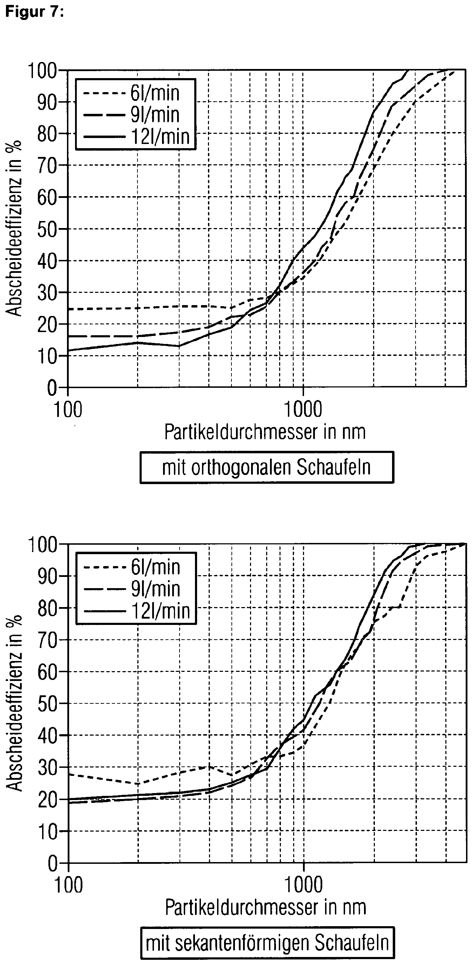

Figur 7 zeigt Trennkurven der in Figur 1 dargestellten Vorrichtungen bei verschiedenen Gasströmen. Zu sehen ist, dass der Abscheidegrad mit dem Partikeldurchmesser und dem Gasvolumenstrom deutlich ansteigt. Partikel > als 1 Mikrometer werden bei Gasströmen zwischen 6 und 12 Liter pro Minute, wie sie für derartige medizintechnische Vorrichtungen üblich sind, bereits zu 10 bis 30 Prozent abgetrennt. Bei Partikeldurchmessern von 2000 bis 3000 Nanometer (entsprechend 2 bis 3 Mikrometer) erfolgt eine Abscheidung unter den gleichen Bedingungen zu mehr als 80 Prozent. Je nach Auslegung der Vorrichtung und Strömungsgeschwindigkeit können auch Partikel mit Größen zwischen 100 und 400 nm abgeschieden werden, welche von Faserfiltern nur sehr schlecht abgeschieden werden. Bei Gasströmen unterhalb von 6 l/min werden Partikel nur noch sehr begrenzt abgetrennt. Gleichwohl kann die erfindungsgemäße Vorrichtung auch bei Gasströmen von 3 - 6 l/min zur Abtrennung von Feuchtigkeit eingesetzt werden.

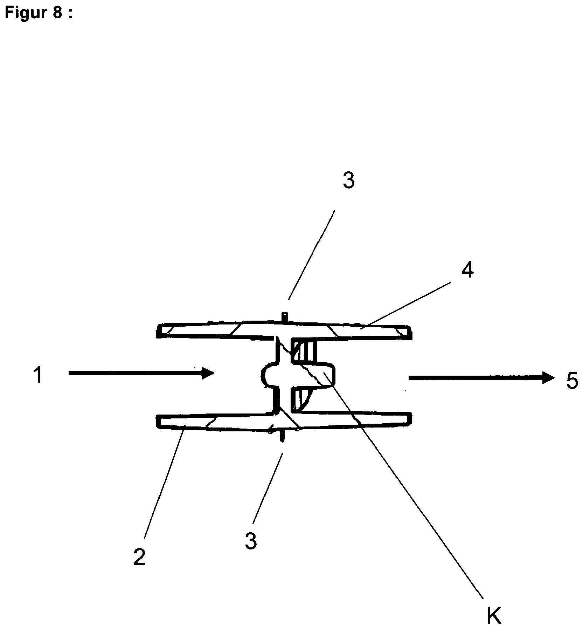

Wie oben bereits dargestellt, besteht die wahrscheinlich einfachste Ausführungsform darin, die erfindungsgemäße Vorrichtung als Schlauchverbinder auszugestalten. Eine Möglichkeit zu einer derartigen Ausgestaltung wird in Figur 4 angedeutet. Alternativ ist, wie in Figur 8 dargestellt, die erfindungsgemäße Vorrichtung eingangsseitig so dimensioniert, dass ein Schlauch leicht aufgesteckt werden kann. Das Gehäuse (2) kann dabei außen leicht konisch zulaufend sein, um ein einfacheres Aufstecken zu ermöglichen. Gegebenenfalls kann der Schlauch durch eine Schlauchschelle gesichert werden. Das Gehäuse kann mit einer Mittelwulst (3) ausgestattet sein. Der Axialkörper (K) mit Leitschaufeln befindet sich im Inneren der Vorrichtung. Auf der Ausgangsseite der Vorrichtung wird ein zweiter Schlauch aufgesteckt und gegebenenfalls ebenfalls durch eine Schlauchschelle gesichert. In jedem Fall wird der aus der medizinischen Absaugvorrichtung (z.B. einer Laparoskopievorrichtung mit Absaugpumpe) kommende Gasstrom durch einen ersten Schlauch der erfindungsgemäßen Vorrichtung zugeführt. Bei Inbetriebnahme der Pumpe wird der Gasstrom durch den ersten Schlauch in die Vorrichtung hinein und durch den zweiten Schlauch wieder hinaus geführt. Der Gasstrom erfolgt in Pfeilrichtung (1,5). Im Gasstrom mitgeführte Partikel und Feuchtigkeit lagert sich im zweiten Schlauch in charakteristischen helixförmigen Bereichen ab. Der zweite Schlauch kann dabei aus einem anderen Material gefertigt sein, als der erste Schlauch, beispielsweise eine höhere Rauigkeit oder eine hydrophilere Oberfläche aufweisen, um die Abscheidung zu begünstigen.

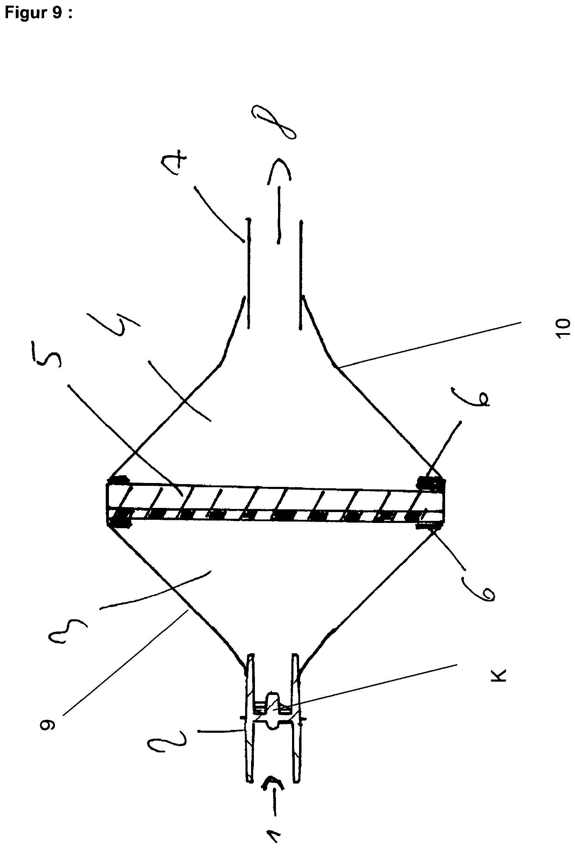

Eine andere Möglichkeit besteht darin, die erfindungsgemäße Vorrichtung in einen Filterhalter zu integrieren (Figur 9). Hierin wird beispielsweise ein Filter (5) in ein Gehäuse eingelegt bzw. eingeklemmt, beispielsweise mit entsprechenden Klemmelementen (6). Das Gehäuse besteht beispielsweis aus zwei kegelförmigen Teilen (9, 10) wobei die beiden Kegelböden beispielsweise durch einen Bajonettverschluss miteinander verbunden sind. Der Filter kann aus zwei Schichten bestehen, wobei zusätzlich zum Faserfilter noch ein Aktivkohlefilter vorgesehen sein kann. Dieser kann flächig mit dem Faserfilter verbunden sein oder rohrförmig in den Gehäuseauslass integriert sein (nicht dargestellt). Im Einlass des Gehäuses ist die erfindungsgemäße Vorrichtung mit dem Axialkörper (K) mit angeformten Leitschaufeln integriert. Der Auslassstutzen der Vorrichtung ist gleichzeitig der Eintritt des Gasstroms in die erste Kammer (3). Nach Durchströmen des Filters (5) wird der Gasstrom dem Gehäuseauslass (7) zugeführt. In dieser Ausführungsform ist die Filtergröße üblicherweise erheblich größer als der Durchmesser des Auslassstutzens, um ein Zusetzen des Filters zu vermeiden. Bei einem Durchmesser des Austrittsstutzens von 20mm empfiehlt es sich eine Filterfläche von 30-100 cm² vorzusehen, wobei es nebensächlich ist, ob die Filterfläche rund, quadratisch, rechteckig oder in anderer Geometrie gestaltet ist.

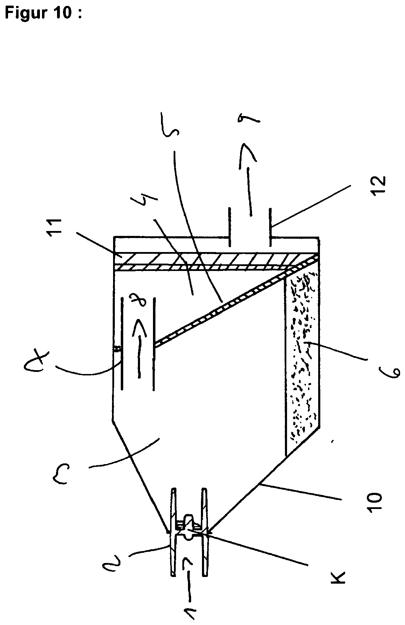

In einer weiteren möglichen Ausführungsform der Erfindung (Figur 10) ist die erfindungsgemäße Vorrichtung in ein Filtergehäuse integriert, welches auch einen Wasserabscheider enthält. Das Gehäuse (10) enthält eine erste Kammer (3) in die der Auslassstutzen der erfindungsgemäßen Vorrichtung (2) mit Axialkörper (K) und Leitschaufeln mündet und somit den Gaseingang in die erste Kammer bildet. Die erste Kammer (3) enthält einen Gasausgang (7). Der Gasausgang (7) kann in einer Linie mit dem Gaseingang liegen. Wie in der Figur 10 dargestellt kann in besonderen Ausführungsformen der Gasausgang auch axial versetzt zum Gaseingang angeordnet sein. Um die gewünschte Wasserabscheidung ohne Wassereintritt in die zweite Kammer (4) zu ermöglichen empfiehlt es sich in diesem Fall den stutzenförmigen Ausgang höher anzuordnen als den Eingang. Der aus der erfindungsgemäßen Vorrichtung kommende Gasstrom trifft auf diese Weise auf die Kammerrückwand (5), auf der sich Feuchtigkeitstropfen abscheiden können. Der Boden der ersten Kammer kann dabei mit feuchtigkeitsabsorbierendem Material (6) (z.B. Superabsorber) ausgelegt sein. Der in die Rückwand integrierte Ausgangsstutzen (7) führt in eine zweite Kammer (4), die mit einer Filteranordnung (11) versehen ist. Die Filteranordnung (11) kann beispielsweise aus einem Faserfilter zur Abscheidung eventuell noch im Gasstrom befindlicher Restpartikel und einem Aktivkohlefilter zur Abscheidung von Gasen (z.B. HCN, CO, SO₂) bestehen. Auch in diesem Fall empfiehlt sich eine großflächige Filteranordnung zur Vermeidung eines Zusetzens der Filter. Der durch diese Gesamtanordnung gefilterte Gasstrom verlässt die zweite Kammer durch einen Ausgangsstutzen (12). Der Gasstrom durch die Gesamtvorrichtung erfolgt in Richtung der Pfeile (1, 8, 9).

Insgesamt ist es mit der Erfindung gelungen, eine einfache Vorrichtung zur Verfügung zu stellen, die kostengünstig herstellbar ist, leicht in bestehende medizinische Anlagen, z.B. medizinische Insufflationsgeräte mit Absaugvorrichtung, integriert werden kann und gleichwohl eine effiziente Abscheidung von Partikeln und Flüssigkeitströpfchen aus Rauchgasen ermöglicht. Die kostengünstige Herstellung erlaubt die Verwendung für eine einzige Anwendung, so dass aufwendige Reinigungs- und Sterilisationsprozesse, wie sie für chirurgische Anwendungen typisch sind, vermieden werden können.

## Patentansprüche

1. Vorrichtung zur Abscheidung von Rauchgaspartikeln, Flüssigkeitströpfchen und Feuchtigkeit aus abgesaugten chirurgischen Gasen im Rahmen laparaskopischer Eingriffe
wobei die Gase eine Strömungsgeschwindigkeit von 3-20 l/min aufweisen, gekennzeichnet
mit einem Rohr der Länge I von 10-50 mm, wobei das Rohr einen Innendurchmesser dᵢ von 4-20 mm aufweist, einem axial gelegenen Strömungskörper K, der einen Spantquerschnitt von 0,5-6 mm aufweist,
mit zwei bis sechs Leitschaufeln L, die zwischen dem axial gelegenen Strömungskörper K und der Innenwand des Rohrs liegen und mit diesen starr verbunden sind,
wobei die Leitschaufeln L helixförmig angeordnet sind, so dass sie dem durchströmenden Gas eine radiale Bewegungskomponente geben.

2. Vorrichtung gemäß Anspruch 1 mit 3-5 Leitschaufeln L.

3. Vorrichtung gemäß Anspruch 1 wobei die Leitschaufeln einen Austrittswinkel β zwischen dem axial gelegenen Strömungskörper K und dem Rohrquerschnitt von 20-30° aufweisen.

4. Vorrichtung gemäß Anspruch 1 wobei das Verhältnis der die Länge lₖ des axialen Strömungskörpers zu dem Spantquerschnitt des axialen Strömungskörper d_{K} zwischen 2 und 3 liegt.

5. Vorrichtung gemäß Anspruch 1 wobei das Verhältnis der die Länge la zwischen der in Strömungsrichtung letzten Kante der Leitschaufel und dem Rohraustritt zu dem Spantquerschnitt des axialen Strömungskörper d_{K} zwischen 2 und 4 liegt.

6. Vorrichtung gemäß Anspruch 1 wobei die Verbindung zwischen axialem Strömungskörper und Leitschaufel orthogonal oder sekantenförmig ist.

7. Vorrichtung gemäß Anspruch 1, wobei das Rohr mit Strömungskörper und Leitschaufeln in ein Gehäuse integriert ist, wobei das Gehäuse noch weitere Vorrichtungen zur Abscheidung von Partikel, Flüssigkeiten und oder Gasen enthält.

8. Vorrichtung gemäß Anspruch 7, wobei Partikel durch ein Faserfilter, Wasser durch einen Superabsorber und/oder Gase durch Aktivkohle abgeschieden werden.

9. Verwendung einer Vorrichtung gemäß mindestens einem der Ansprüche 1-8 in medizintechnischen Vorrichtungen zur Abscheidung von Rauchgaspartikeln, Flüssigkeitströpfchen und Feuchtigkeit aus abgesaugten chirurgischen Gasen im Rahmen laparaskopischer Eingriffe.

## Claims

1. A device for the separation of flue gas particles, liquid droplets, and humidity from extracted surgical gases during laparoscopic interventions,
wherein the gases have a flow rate of 3 to 20 1/min,
**characterized by** a tube of length 1 of 10 to 50 mm, the tube having an inner diameter dᵢ of 4 to 20 mm,
an axially located flow body K having a web cross-section of 0.5 to 6 mm,
two to six guide vanes L disposed between the axially located flow body K and the inner wall of the tube and being rigidly connected thereto,
wherein the guide vanes L are helically arranged to confer a radial movement component to the gas flowing therethrough.

2. The device according to claim 1 comprising 3 to 5 guide vanes L.

3. The device according to claim 1, wherein the guide vanes have an exit angle β between the axially located flow body K and the tube cross-section of 20 to 30 degrees.

4. The device according to claim 1, wherein the ratio of the length lₖ of the axial flow body to the web cross-section d_{K} of the axial flow body is between 2 and 3.

5. The device according to claim 1, wherein the ratio of the length lₐ between the last edge of the guide vane, in the direction of flow, and the tube exit to the web cross-section d_{K} of the axial flow body is between 2 and 4.

6. The device according to claim 1, wherein the connection between the axial flow body and the guide vane is orthogonal or secant-shaped.

7. The device according to claim 1, wherein the tube with flow body and guide vanes is integrated in a housing, the housing comprising further devices for the separation of particles, liquids, and/or gases.

8. The device according to claim 7, wherein particles are separated by a fiber filter, water is separated by a superabsorber, and/or gases are separated by activated carbon.

9. Use of a device according to at least one of claims 1 to 8 for the separation of flue gas particles, liquid droplets, and humidity from extracted surgical gases during laparoscopic interventions.

## Revendications

1. Dispositif pour la séparation de particules de gaz de combustion, gouttelettes de liquide, et humidité à partir de gaz chirurgicaux aspirés pendant des interventions laparoscopiques,
dans lequel le gaz a un débit de 3 à 20 l/min, **caractérisé par** un tuyau de longueur l de 10 à 50 mm, le tuyau ayant un diamètre intérieur dᵢ de 4 à 20 mm,
un corps d'écoulement K positionné axialement et ayant une section transversale de cadre de 0,5 à 6 mm,
deux à six aubes de guidage L disposées entre le corps d'écoulement K positionné axialement et la paroi intérieure du tuyau et étant liées rigidement à celui-ci,
dans lequel les aubes de guidage L sont arrangées en hélice à donner un composant de mouvement radial au gaz traversant.

2. Dispositif selon la revendication 1 comprenant 3 à 5 aubes de guidage L.

3. Dispositif selon la revendication 1, dans lequel les aubes de guidage ont un angle de sortie β entre le corps d'écoulement K positionné axialement et la section transversale du tuyau de 20 à 30 degrés.

4. Dispositif selon la revendication 1, dans lequel le rapport entre la longueur lₖ du corps d'écoulement axial et la section transversale de cadre d_{K} du corps d'écoulement axial est compris entre 2 et 3.

5. Dispositif selon la revendication 1, dans lequel le rapport entre la longueur lₐ et l'arête dernière de l'aube de guidage, dans la direction d'écoulement, et la sortie du tuyau à la section transversale de cadre d_{K} du corps d'écoulement axial est compris entre 2 et 4.

6. Dispositif selon la revendication 1, dans lequel la liaison entre le corps d'écoulement axial et l'aube de guidage est orthogonale ou en forme de sécante.

7. Dispositif selon la revendication 1, dans lequel le tuyau avec corps d'écoulement et aubes de guidage est intégré dans un boîtier, le boîtier comprenant d'autres dispositifs pour la séparation de particules, liquides et/ou gaz.

8. Dispositif selon la revendication 7, dans lequel des particules sont séparées par un filtre à fibres, de l'eau est séparée par un superabsorbeur et/ou du gaz est séparé par des charbons actifs.

9. Utilisation d'un dispositif selon au moins une des revendications 1 à 8 pour la séparation de particules de gaz de combustion, gouttelettes de liquide et humidité à partir de gaz chirurgicaux aspirés pendant des interventions laparoscopiques.
